# EUROPEAN PATENT SPECIFICATION

(11) **EP 2 660 282 B1**
(45) Date of publication and mention of the grant of the patent: **27.09.2017**
(21) Application number: 13166420.3
(22) Date of filing: 26.06.2006
(51) Int. Cl.: B01J 37/03, C08J 9/14, C09K 5/04, C10M 171/00

(54) **Thermoset foam comprising HFCO-1233zd as blowing agent**
Wärmehärtender Schaumstoff der HFCO-1233zd als Treibmittel enthält
Mousse thermodurcissable comprenant HFCO-1233zd comme agent d'expansion

(30) Priority: 24.06.2005 US 693853 P; 21.03.2006 US 784731 P
(43) Date of publication of application: 06.11.2013
(62) Divisional of application: 06785686.4
(73) Proprietor: Honeywell International Inc., Morris Plains, NJ 07950 (US)
(72) Inventor: Bowman, James M., Geneva, IL Illinois 60134 (US); Williams, David J., East Amherst, NY New York 14051 (US)
(74) Representative: Crooks, Elizabeth Caroline

(56) References cited:
- EP-B1- 2 154 223
- US-A- 5 786 401
- US-A1- 2004 256 594
- US-A1- 2005 085 674
- US-A1- 2005 131 091
- AOYAMA H ET AL: "Foaming agent of fluoro:propene for producing foams - comprises tri-fluoro:methyl-tri:fluoro-propane, giving no ozone layer depletion having excellent dimensional stability, etc", WPI / THOMSON,, vol. 1993, no. 33, 20 July 1993 (1993-07-20), XP002719442,

## Description

### FIELD OF THE INVENTION

This invention relates to compositions, methods and systems having utility in the production of a thermoset foam.

### BACKGROUND

Fluorocarbon based fluids have found widespread use in many commercial and industrial applications, including as aerosol propellants and as blowing agents. Because of certain suspected environmental problems, including the relatively high global warming potentials, associated with the use of some of the compositions that have heretofore been used in these applications, it has become increasingly desirable to use fluids having low or even zero ozone depletion potential, such as hydrofluorocarbons ("HFCs"). Thus, the use of fluids that do not contain substantial amounts of chlorofluorocarbons ("CFCs") or hydrochlorofluorocarbons ("HCFCs") is desirable. Furthermore, some HFC fluids may have relatively high global warming potentials associated therewith, and it is desirable to use hydrofluorocarbon or other fluorinated fluids having as low global warming potentials as possible while maintaining the desired performance in use properties. Additionally, the use of single component fluids or azeotrope-like mixtures, which do not substantially fractionate on boiling and evaporation, is desirable in certain circumstances.

As suggested above, concern has been increasing in recent years about potential damage to the earth's atmosphere and climate, and certain chlorine-based compounds have been identified as particularly problematic in this regard. The use of chlorine-containing compositions (such as chlorofluorocarbons (CFC's), and hydrochlorofluorocarbons (HCF's)) as the working fluid in heat transfer systems, such as in refrigeration and air-conditioning systems, has become disfavored because of the ozone-depleting properties associated with many of such compounds. There has thus been an increasing need for new fluorocarbon and hydrofluorocarbon compounds and compositions that are attractive alternatives to the compositions heretofore used in these and other applications. For example, it has become desirable to retrofit chlorine-containing systems, such as blowing agent systems or refrigeration systems, by replacing chlorine-containing compounds with non-chlorine-containing compounds that will not deplete the ozone layer, such as hydrofluorocarbons (HFC's). Industry in general is continually seeking new fluorocarbon based mixtures that offer alternatives to, and are considered environmentally safer substitutes for, CFCs and HCFCs. It is considered important in many cases, however, that any potential substitute must also possess those properties present in many of the most widely used fluids, such as imparting excellent thermal insulating properties and other desirable foam characteristics when used as blowing agents, such as appropriate chemical stability, low- or no- toxicity, low or no-flammability, among others.

Furthermore, it is generally considered desirably for CFC blowing agent substitutes to be effective without major engineering changes to conventional foam generating systems.

Methods and compositions for making conventional foamed materials, such as for example thermoplastic materials and thermosetting materials, have long been known. These methods and compositions have typically utilized chemical and/or physical blowing agents to form the foamed structure in a polymeric matrix. Such blowing agents have included, for example, azo compounds, various volatile organic compounds (VOCs) and chlorofluorocarbons (CFCs). The chemical blowing agents typically undergo some form of chemical change, including chemical reaction with the material that forms the polymer matrix (usually at a predetermined temperature/pressure) that causes the release of a gas, such as nitrogen, carbon dioxide, or carbon monoxide. One of the most frequently used chemical blowing agents is water. The physical blowing agents typically are dissolved in the polymer or polymer
precursor material and then expand volumetrically (again at a predetermined temperature/pressure) to contribute to the formation of the foamed structure. Physical blowing agents are frequently used in connection with thermoplastic foams, although chemical blowing agents can be used in place of or in addition to physical blowing agents in connection with thermoplastic foam. For example, it is known to use chemical blowing agent in connection with the formation of polyvinylchloride-based foams. It is common to use chemical blowing and/or physical blowing agents in connection with thermosetting foams. Of course, it is possible that certain compounds and the compositions that contain them may at once constitute a chemical and a physical blowing agent.

It was common in the past that the CFCs were used as standard blowing agents in the preparation of isocyanate-based foams, such as rigid and flexible polyurethane and polyisocyanurate foams. For example, CCl₃F (CFC-11) had become a standard blowing agent. However, the use of this material has been banned by international treaty on the grounds that its release into the atmosphere damages the ozone layer in the stratosphere. As a consequence, it is no longer generally common that neat CFC-11 is used as a standard blowing agent for forming thermosetting foams, such as isocyanate-based foams and phenolic foams.

The problems with CFCs led to the more frequent utilization hydrogencontaining chlorofluoroalkanes (HCFCs). For example, CHCl₂CF₃ (HCFC-123), CH₂ClCHClF (HCFC-141b) have relatively short lifetimes in the atmosphere. However, while HCFCs are considered to be environmentally friendly blowing agents relative to CFCs, such compounds still contain some chlorine, and therefore have an "Ozone Depletion Potential" (called "ODP"). Because of the non-zero ODP, HCFCs have been targeted for eventual removal from use.

Another known class of blowing agents is the non-chlorinated, partially hydrogenated fluorocarbons (called "HFCs"). Certain of the HFC currently being used as blowing agents have at least one potentially serious problem, namely that they generally have relatively high intrinsic thermal conductivity properties (i.e., poor thermal insulation). On the other hand, foams made with certain of the more modern HFC blowing agents, such as CF₃CH₂CF₂H ("HFC-245fa") offer improved thermal insulation,
due in part to the low thermal conductivity of HFC-245fa vapor, and due in part to the fine cell structure HFC-245fa imparts to the foams. HFC-245fa has been widely used in insulation applications, particularly refrigerator, freezer, refrigerator/freezer and spray foam applications. Nevertheless, many HFC fluids share the disadvantage of having relatively high global warming potentials, and it is desirable to use hydrofluorocarbon or other fluorinated fluids having as low global warming potentials as possible while maintaining the desired performance in use properties. Even the more modern HFCs, such as HFC-245fa, HFC-134a, HFC-365mfc, and others, exhibit a higher than desirable global warming potential, albeit low relative to other HFCs. Thus, the use of HFCs as blowing agents in foam insulation, particularly rigid foam insulation, has resulted in HFCs being less desirable candidates for blowing agents in commercial foam insulation.

Hydrocarbon blowing agents are also known. For example, U.S. Pat. No. 5,182,309 to Hutzen teaches the use of iso- and normal-pentane in various emulsion mixtures. Another example of hydrocarbon blowing agents is cyclopentane, as taught by U.S. Pat. No. 5,096,933- Volkert. Although many hydrocarbon blowing agents, such as cyclopentane, and isomers of pentane, are zero ozone depleting agents and exhibit very low global warming potential, such material are less than fully desirable because foams produced from these blowing agents lack the same degree of thermal insulation efficiency as foams made with, for example, HFC-245fa blowing agent. Further, the hydrocarbon blowing agents are extremely flammable, which is undesirable. Also, certain hydrocarbon blowing agents have inadequate miscibility in certain situations with material from which the foam is formed, such as many of the polyester polyols commonly used in polyisocyanurate modified polyurethane foam. The use of these alkanes frequently requires a chemical surfactant to obtain a suitable mixture.

There has thus been an increasing need for new compounds and compositions that are attractive alternatives to the compositions heretofore used as blowing agents in these and other applications. Applicants have thus recognized a need for new fluorocarbon based compounds and compositions that offer effective alternatives to, and are considered environmentally safer substitutes for, CFCs and HCFCs. It is generally considered highly desirable, however, that any potential substitute must also
possess properties, or impart properties to the foam, that are at least comparable to those associated with many of the most widely used blowing agents, such as vapor phase thermal conductivity (low k-factor), low- or no- toxicity, among others.

One such other potentially important property in many applications is flammability. That is, it is considered either important or essential in many applications, including particularly in blowing agent applications, to use compositions which are of low flammability or are non-flammable. As used herein, the term "nonflammable" refers to compounds or compositions which are determined to be nonflammable as determined in accordance with ASTM standard E-681, dated 2002. Unfortunately, many HFC's which might otherwise be desirable for used in refrigerant compositions are not nonflammable. For example, the fluoroalkane difluoroethane (HFC-152a) and the fluoroalkene 1,1,1-trifluorpropene (HFO-1243zf) are each flammable and therefore not viable for use in many applications.

It has been suggested to use bromine-containing halocarbon additives to decrease flammability of certain materials, including foam blowing agents, in U.S. Patent 5,900,185 - Tapscott. The additives in this patent are said to be characterized by high efficiency and short atmospheric lifetimes, that is, low ozone depletion potential (ODP) and a low global warming potential (GWP).

While the brominated olefins described in Tapscott may have some level of effectiveness as anti-flammability agents in connection with certain materials, there is no disclosure of the use of such materials as a blowing agent. Furthermore, it is believed that such compounds may also have certain disadvantages. For example, applicants have come to recognize that many of the compounds identified in Tapscott will have a relatively low efficiency as a blowing agent due to the relatively high molecular weight of such compounds. In addition, it is believed that many of the compounds disclosed in Tapscott will encounter problems when used as a blowing agent due to the relatively high boiling point of such compounds. Moreover, it is understood by applicants that many compounds which have a high level of substitution may possess undesirable toxicity properties and/or other undesirable properties, such as potentially environmentally undesirable bioaccumulation.

While Tapscott indicates that bromine-containing alkenes having from 2 to 6 carbon atoms may also contain fluorine substituents, this patent appears to suggest that fluorine-containing compounds are less than fully desirable from the standpoint of environmental safety by noting that "non-fluorine-containing bromoalkanes will have very short atmospheric lifetimes due to reaction with tropospheric hydroxyl free radicals." (Col. 8, I. 34 - 39).

Furthermore, it is generally considered desirable for blowing agent substitutes to be effective without major engineering changes to conventional equipment and systems used in foam preparation and formation.

Applicants have thus come to appreciate a need for compositions, and particularly blowing agents, foamable compositons, foamed articles and methods and systems for forming foam, which provide beneficial properties and/or avoid one or more of the disadvantages noted above. Applicants have thus come to appreciate a need for compositions, and particularly blowing agents, that are potentially useful in numerous applications, while avoiding one or more of the disadvantages noted above.

This invention relates to compositions, methods and systems having utility in numerous applications, including particularly in connection with compositions, methods, systems and agents relating to polymeric thermoset foams.

### SUMMARY

Applicants have found that the above-noted need, and other needs, can be satisfied by blowing agent compositions, foamable compositions, foams and/or foamed articles comprising 1-chloro-3,3,3-trifluoropropene (HFCO-1233zd).

The term HFCO-1233zd is used herein generically to refer to 1,1,1-trifluo-3,chloro-propene, independent of whether it is the cis- or trans- form. The terms "cisHFCO-1233zd" and "transHFCO-1233zd" are used herein to describe the cis-and trans- forms of 1, 1, 1-trifluo,3-chlororopropene, respectively. The term "HFCO-1233zd" therefore includes within its scope cisHFCO-1233zd, transHFCO-1233zd, and all combinations and mixtures of these.

The present invention provides also methods and systems which utilize the compositions of the present invention, including methods and systems for foam blowing.

The present invention provides a thermoset foam as set out in claim 1. The invention further provides the use of the foam as set out in claim 19, a method of manufacturing the foam as set out in claim 20 and a product comprising the foam as set out in claim 21.

The invention provides a thermoset foam comprising a plurality of polymeric cells and a blowing agent composition contained in at least one of said cells, said composition comprising HFCO-1233zd (1-chloro-3,3,3-trifluoropropene), wherein the foam has a K factor in mW.m⁻¹K⁻¹ (BTU in/hr ft² °F) at 4°C (40 °F) of not greater than 20.2 (0.14)
wherein the HFCO-1233zd is present in the composition in an amount of at least 5% by weight of the composition and
wherein the composition has a Global Warming Potential (GWP) of not greater than 500.

The invention provides the use of a foam described above as an appliance foam selected from refrigerator foams, freezer foams, refrigerator/freezer foams, and panel foams.

The invention provides a method of providing a thermoset foam as described above, said method (a) providing an A side component comprising an isocyanate and a B side component comprising a polyol or polyol mixture, a surfactant, a catalyst, a flame retardant and a composition comprising at least 5% by weight of HFCO-1233zd (1-chloro-3,3,3-trifluoropropene) and (b) mixing the A and B side components by hand mixing or machine mixing to form a foam.

The invention provides a product selected from blocks, slabs, laminates, pour-in-place panels, spray-applied foam and froths, wherein said product comprises a foam obtainable from the above method.

### DETAILED DESCRIPTION OF PREFERRED EMBODIMENTS

### THE COMPOSITIONS

The present compositions can generally be in the form of blowing agent compositions or foamable compositions. In each case, the present invention requires at least HFCO-1233zd as described herein and optionally other ingredients, some of which are described in detail below.

Applicants believe that HFCO-1233zd is generally effective and exhibits utility in blowing agent compositions in accordance with the teachings contained herein. However, applicants have surprisingly and unexpectedly found that HFCO-1233zd exhibits a highly desirable low level of toxicity. As can be readily appreciated, this discovery is of potentially enormous advantage and benefit for the formulation of blowing agent compositions.

The present compositions comprising HFCO-1233zd, are believed to possess properties that are advantageous for a number of important reasons. For example, applicants believe, based at least in part on mathematical modeling, that the fluoroolefin used in the present invention will not have a substantial negative affect on atmospheric chemistry, being negligible contributors to ozone depletion in comparison to some other halogenated species. The preferred compositions of the present invention thus have the advantage of not contributing substantially to ozone depletion. The preferred compositions also do not contribute substantially to global warming compared to many of the hydrofluoroalkanes presently in use.

In certain preferred forms, compositions of the present invention have a Global Warming Potential (GWP) of not greater than 1000, more preferably not greater than 500, and even more preferably not greater than 150. In certain embodiments, the GWP of the present compositions is not greater than about 100 and even more preferably not greater than 75. As used herein, "GWP" is measured relative to that of carbon dioxide and over a 100 year time horizon, as defined in "The Scientific Assessment of Ozone Depletion, 2002, a report of the World Meteorological Association's Global Ozone Research and Monitoring Project,".

In certain preferred forms, the present compositions also preferably have an Ozone Depletion Potential (ODP) of not greater than 0.05, more preferably not greater
than 0.02 and even more preferably zero. As used herein, "ODP" is as defined in "The Scientific Assessment of Ozone Depletion, 2002, A report of the World Meteorological Association's Global Ozone Research and Monitoring Project,".

The amount of the HFCO-1233zd, contained in the present compositions can vary widely, depending the particular application. Moreover, the compositions of the present invention can be azeotropic, azeotrope-like or non-azeotropic. The blowing agent composition, can comprise HFCO-1233zd in amounts from 5% to 95% by weight, and more preferably from 40% to 90% by weight.

### B. OTHER COMPONENTS - BLOWING AGENT COMPOSITIONS

It is contemplated that in certain embodiments of the present invention the blowing agent compositions consist of or consist essentially of HFCO-1233zd. Thus, the present invention includes methods and systems which include HFCO-1233zd as a blowing agent without the presence of any substantial amount of additional components. However, one or more compounds or components that are not HFCO-1233zd are optionally, but preferably, included in the blowing agent compositions of the present invention. Such optional additional compounds include, but are not limited to, other compounds which also act as blowing agents (hereinafter referred to for convenience but not by way of limitation as co-blowing agents), surfactants, polymer modifiers, toughening agents, colorants, dyes, solubility enhancers, rheology modifiers, plasticizing agents, flammability suppressants, antibacterial agents, viscosity reduction modifiers, fillers, vapor pressure modifiers, nucleating agents and catalysts. In certain preferred embodiments, dispersing
agents, cell stabilizers, surfactants and other additives may also be incorporated into the blowing agent compositions of the present invention. Certain surfactants are optionally but preferably added to serve as cell stabilizers. Some representative materials are sold under the names of DC-193, B-8404, and L-5340 which are, generally, polysiloxane polyoxyalkylene block co-polymers such as those disclosed in U.S. Patent Nos. 2,834,748, 2,917,480, and 2,846,458. Other optional additives for the blowing agent mixture may include flame retardants such as tri(2-chloroethyl)phosphate, tri(2-chloropropyl)phosphate, tri(2,3-dibromopropyl)-phosphate, tri(1,3-dichloropropyl) phosphate, diammonium phosphate, various halogenated aromatic compounds, antimony oxide, aluminum trihydrate and polyvinyl chloride.

With respect to nucleating agents, all known compounds and materials having nucleating functionality are available for use in the present invention, including particularly talc.

Of course other compounds and/or components that modulate a particular property of the compositions (such as cost for example) may also be included in the present compositions, and the presence of all such compounds and components is within the broad scope of the invention.

Thus, the preferred embodiments of the present compositions include, in addition to HFCO-1233zd, one or more co-blowing agents. The co-blowing agent in accordance with the present invention can comprise a physical blowing agent, a chemical blowing agent (which preferably in certain embodiments comprises water) or a blowing agent having a combination of physical and chemical blowing agent properties. It will also be appreciated that the blowing agents included in the present compositions, including HFCO-1233zd as well as the co-blowing agent, may exhibit properties in addition to those required to be characterized as a blowing agent. For example, it is contemplated that the blowing agent compositions of the present invention may include components, including HFCO-1233zd, which also impart some beneficial property to the blowing agent composition or to the foamable composition to which it is added. For example, it is within the scope of the present
invention for the HFCO-1233zd or for the co-blowing agent to also act as a polymer modifier or as a viscosity reduction modifier.

Although it is contemplated that a wide range of co-blowing agents may be used in accordance with the present invention, in certain embodiments it is preferred that the blowing agent compositions of the present invention include one or more HFCs as co-blowing agents, more preferably one or more C1-C4 HFCs, and/or one or more hydrocarbons, more preferably C4 - C6 hydrocarbons. For example, with respect to HFCs, the present blowing agent compositions may include one or more of difluoromethane (HFC-32), fluoroethane (HFC-161), difluoroethane (HFC-152), trifluoroethane (HFC-143), tetrafluoroethane (HFC-134), pentafluoroethane (HFC-125), pentafluoropropane (HFC-245), hexafluoropropane (HFC-236), heptafluoropropane (HFC-227ea), pentafluorobutane (HFC-365), hexafluorobutane (HFC-356) and all isomers of all such HFC's. With respect to hydrocarbons, the present blowing agent compositions may include in certain preferred embodiments, for example, iso, normal and/or cyclopentane for thermoset foams. Of course other materials, such as water, CO₂, CFCs (such as trichlorofluoromethane (CFC-11) and dichlorodifluoromethane (CFC-12)), hydrochlorocarbons (HCCs such as dichloroethylene (preferably trans-dichloroethylene), ethyl chloride and chloropropane), HCFCs, Cl - C5 alcohols (such as, for example, ethanol and/or propanol and/or butanol), Cl - C4 aldehydes, C3 - C4 ketones, C2- C4 ethers (including ethers (such as dimethyl ether and diethyl ether), diethers (such as dimethoxy methane and diethoxy methane)), and methyl formate including combinations of any of these may be included, although such components are contemplated to be not preferred in many embodiments due to negative environmental impact.

In certain embodiments, one or more of the following HFC isomers are preferred for use as co-blowing agents in the compositions of the present invention:
1,1,1,2,2-pentafluoroethane (HFC-125)
1,1,2,2-tetrafluoroethane (HFC-134)
1,1,1,2-tetrafluoroethane (HFC-134a)
1,1- difluoroethane (HFC-152a)
1,1,1,2,3,3,3-heptafluoropropane (HFC-227ea)
1,1,1,3,3,3-hexafluoropropane (HFC-236fa)
1,1,1,3,3-pentafluoropropane (HFC-245fa) and
1,1,1,3,3-pentafluorobutane (HFC-365mfc).

The relative amount of any of the above noted additional co-blowing agents, as well as any additional components which may be included in present compositions, can vary widely within the general broad scope of the present invention according to the particular application for the composition, and all such relative amounts are considered to be within the scope hereof. Applicants note, however, that one particular advantage of HFCO-1233zd is the relatively low flammability of such compounds. Accordingly, in certain embodiments it is preferred that the blowing agent composition of the present invention comprise at least one co-blowing agent and an amount of HFCO-1233zd sufficient to produce a blowing agent composition which is overall nonflammable. Thus, in such embodiments, the relative amounts of the co-blowing agent in comparison to the HFCO-1233zd will depend, at least in part, upon the flammability of the co-blowing agent.

The blowing agent compositions of the present invention may include the compounds of the present invention in widely ranging amounts. It is generally preferred, however, that for preferred compositions for use as blowing agents in accordance with the present invention, HFCO-1233zd is present in an amount that is at least 1 % by weight, more preferably at least 5 % by weight, and more preferably at least 15 % by weight, of the composition. In certain preferred embodiments, the blowing agent comprises at least 50% by weight of the present blowing agent compound(s), and in certain embodiments the blowing agent consists essentially of HFCO-1233zd. In this regard it is noted that the use of one or more co-blowing agents is consistent with the novel and basic features of the present invention. For example, it is contemplated that water will be used as either a co-blowing or in combination with other co-blowing agents (such as, for example, pentane, particularly cyclopentane) in a large number of embodiments.

In many preferred embodiments, a co-blowing agent comprising water is included in the compositions directed to the use of thermosetting foams.

In certain preferred embodiments, the blowing agent composition comprises from 30% to 95 % by weight of HFCO-1233zd, and 5% to 90% by weight, more preferably from 5% to 65% by weight of co-blowing agent. In certain of such embodiments the co-blowing agent comprises, and preferably consists essentially of, H₂O, HFCs, hydrocarbons, alcohols (preferably C2, C3 and/or C4 alcohols), CO₂, and combinations of these.

In preferred embodiments in which the co-blowing agent comprises H₂O, the composition comprises H₂O in an amount of from 5% by weight to 50% by weight of the total blowing agent composition, more preferably from 10% by weight to 40% by weight, and even more preferably of from 10% to 20% by weight of the total blowing agent.

In preferred embodiments in which the co-blowing agent comprises CO₂, the composition comprises CO₂ in an amount of from 5% by weight to 60% by weight of the total blowing agent composition, more preferably from 20% by weight to 50% by weight, and even more preferably of from 40% to 50% by weight of the total blowing agent.

In preferred embodiments in which the co-blowing agent comprises alcohols, (preferably C2, C3 and/or C4 alcohols), the composition comprises alcohol in an amount of from 5% by weight to 40% by weight of the total blowing agent composition, more preferably from 10% by weight to 40% by weight, and even more preferably of from 15% to 25% by weight of the total blowing agent.

For compositions which include HFC co-blowing agents, the HFC co-blowing agent (preferably C2, C3, C4 and/or C5 HFC), and even more preferably difluoromethane (HFC-152a) (HFC-152a being particularly preferred for extruded thermoplastics) and/or pentafluoropropane (HFC-245)), is preferably present in the composition in amounts of from of from 5% by weight to 80% by weight of the total blowing agent composition, more preferably from 10% by weight to 75% by weight, and even more preferably of from 25% to 75% by weight of the total blowing agent. Furthermore, in such embodiments, the HFC is preferably C2-C4 HFC, and even more preferably C3 HFC, with penta-fluorinated C3 HFC, such as HFC-245fa, being highly preferred in certain embodiments.

For compositions which include HC co-blowing agents, the HC co-blowing agent (preferably C3, C4 and/or C5 HC) is preferably present in the composition in amounts of from 5% by weight to 80% by weight of the total blowing agent composition, and even more preferably from 20% by weight to 60% by weight of the total blowing agent.

### C. OTHER COMPONENTS -- FOAMABLE COMPOSITIONS

As is known to those skilled in the art, foamable compositions generally include one or more components capable of forming foam. As used herein, the term "foam foaming agent" is used to refer to a component, or a combination on components, which are capable of forming a foam structure, preferably a generally cellular foam structure. The foamable compositions of the present invention include such component(s) and HFCO-1233zd

The one or more components capable of forming foam comprise a thermosetting composition capable of forming foam and/or foamable compositions. Examples of thermosetting compositions include polyurethane and polyisocyanurate foam compositions, and also phenolic foam compositions. This reaction and foaming process may be enhanced through the use of various additives such as catalysts and surfactant materials that serve to control and adjust cell size and to stabilize the foam structure during formation. Furthermore, is contemplated that any one or more of the additional components described above with respect to the blowing agent compositions of the present invention could be incorporated into the foamable composition of the present invention. In such thermosetting foam embodiments, one or more of the present compositions are included as or part of a blowing agent in a foamable composition, or as a part of a two or more part foamable composition, which preferably includes one or more of the components capable of reacting and/or foaming under the proper conditions to form a foam or cellular structure.

### METHODS AND SYSTEMS

It is contemplated that all presently known and available methods and systems for forming foam are readily adaptable for use in connection with the present invention. For example, the methods of the present invention generally require incorporating a blowing agent in accordance with the present invention into a foamable or foam forming
composition and then foaming the composition, preferably by a step or series of steps which include causing volumetric expansion of the blowing agent in accordance with the present invention. In general, it is contemplated that the presently used systems and devices for incorporation of blowing agent and for foaming are readily adaptable for use in accordance with the present invention. In fact, it is believed that one advantage of the present invention is the provision of an improved blowing agent which is generally compatible with existing foaming methods and systems.

Thus, it will be appreciated by those skilled in the art that the present invention comprises methods and systems for foaming thermosetting foams,. Thus, one aspect of the present invention is the use of the present blowing agents in connection conventional foaming equipment, such as polyurethane foaming equipment, at conventional processing conditions. The present methods therefore include masterbatch type operations, blending type operations, third stream blowing agent addition, and blowing agent addition at the foam head.

The present invention relates to methods of forming foams, and preferably polyurethane and polyisocyanurate foams. The methods generally comprise providing a blowing agent composition of the present inventions, adding (directly or indirectly) the blowing agent composition to a foamable composition, and reacting the foamable composition under the conditions effective to form a foam or cellular structure, as is well known in the art. Any of the methods well known in the art, such as those described in "Polyurethanes Chemistry and Technology," Volumes I and II, Saunders and Frisch, 1962, John Wiley and Sons, New York, NY, may be used or adapted for use in accordance with the foam embodiments of the present invention. In general, such preferred methods comprise preparing polyurethane or polyisocyanurate foams by combining an isocyanate, a polyol or mixture of polyols, a blowing agent or mixture of blowing agents comprising one or more of the present compositions, and other materials such as catalysts, surfactants, and optionally, flame retardants, colorants, or other additives.

It is convenient in many applications to provide the components for polyurethane or polyisocyanurate foams in pre-blended formulations. Most typically, the foam formulation is pre-blended into two components. The isocyanate and optionally certain surfactants and blowing agents comprise the first component, commonly referred to as the "A" component. The polyol or polyol mixture, surfactant, catalysts, blowing agents, flame retardant, and other isocyanate reactive components comprise the second
component, commonly referred to as the "B" component. Accordingly, polyurethane or polyisocyanurate foams are readily prepared by bringing together the A and B side components either by hand mix for small preparations and, preferably, machine mix techniques to form blocks, slabs, laminates, pour-in-place panels and other items, spray applied foams and froths. Optionally, other ingredients such as fire retardants, colorants, auxiliary blowing agents, and even other polyols can be added as one or more additional streams to the mix head or reaction site. Most preferably, however, they are all incorporated into one B-component as described above.

The present methods and systems also include forming a one component foam, preferably polyurethane foam, containing a blowing agent in accordance with the present invention. In certain preferably embodiments, a portion of the the blowing agent is contained in the foam forming agent, preferably by being dissolved in a foam forming agent which is liquid at the pressure within the container, a second portion of the blowing agent is present as a separate gas phase. In such systems, the contained/dissolved blowing agent performs, in large part, to cause the expansion of the foam, and the separate gas phase operates to impart propulsive force to the foam forming agent. Such one component systems are typically and preferably packaged in a container, such as an aerosol type can, and the blowing agent of the present invention thus preferably provides for expansion of the foam and/or the energy to transport the foam/foamable material from the package, and preferably both. In certain embodiments, such systems and methods comprise charging the package with a fully formulated system (preferably isocyanate/polyol system) and incorporating a gaseous blowing agent in accordance with the present invention into the package, preferably an aerosol type can.

Any of the methods well known in the art, such as those described in "Polyurethanes Chemistry and Technology," Volumes I and II, Saunders and Frisch, 1962, John Wiley and Sons, New York, NY, may be used or adapted for use in accordance with the foam forming embodiments of the present invention.

It is contemplated also that in certain embodiments it may be desirable to utilize the present compositions when in the supercritical or near supercritical state as a blowing agent.

### THE FOAMS

The invention also relates to all foams, prepared from a polymer foam formulation containing a blowing agent comprising the compositions of the invention. Applicants have found that one advantage of the thermoset foams, such as polyurethane foams, in accordance with the present invention is the ability to achieve, exceptional thermal performance, such as can be measured by the K-factor or lambda, particularly and preferably under low temperature conditions. Although it is contemplated that the present foams, particularly thermoset foams of the present invention, may be used in a wide variety of applications, in certain preferred embodiments the present invention comprises appliance foams in accordance with the present invention, including refrigerator foams, freezer foams, refrigerator/freezer foams, panel foams, and other cold or cryogenic manufacturing applications.

The thermoset foams in accordance with the present invention, in certain preferred embodiments, provide one or more exceptional features, characteristics and/or properties, including: thermal insulation efficiency, dimensional stability, compressive strength, aging of thermal insulation properties, all in addition to the low ozone depletion potential and low global warming potential associated with many of the preferred blowing agents of the present invention. In certain highly preferred embodiments, the present invention provides thermoset foam, including such foam formed into foam articles, which exhibit improved thermal conductivity relative to foams made using the same blowing agent (or a commonly used blowing agent HFC-245fa) in the same amount but without the compound of Formula I in accordance with the present invention. In certain highly preferred embodiments, the thermoset foams, and preferably polyurethane foams, of the present invention exhibit in mW.m⁻¹K⁻¹ (K-factor (BTU in / hr ft²°F)) at 4°C (40°F) of not greater than 20.2 (0.14), more preferably not greater than 19.5 (0.135), and even more preferably not greater than 18.7 (0.13). Furthermore, in certain embodiments, it is preferred that the thermoset foams, and preferably the polyurethane foams of the present invention exhibit a K-factor in mW.m⁻¹K⁻¹ ((BTU in / hr ft² °F)) at 24°C (75°F) of not greater than 23.1 (0.16), more preferably not greater than 21.6 (0.15), and even more preferably not greater than 20.9 (0.145).

In other preferred embodiments, the present foams exhibit improved mechanical properties relative to foams produced with blowing agents outside the scope of the present invention. For example, certain preferred embodiments of the present invention provide foams and foam articles having a compressive strength which is superior to, and preferably at least 10 relative percent, and even more preferably at least 15 relative percent greater than a foam produced under substantially identical conditions by utilizing a blowing agent consisting of cyclopentane. Furthermore, it is preferred in certain embodiments that the foams produced in accordance with the present invention have compressive strengths that are on a commercial basis comparable to the compressive strength produced by making a foam under substantially the same conditions except wherein the blowing agent consists of HFC-245fa. In certain preferred embodiments, the foams of the present invention exhibit a compressive strength of at least 12.5% yield (in the parallel and perpendicular directions), and even more preferably at least 13% yield in each of said directions.

### EXAMPLES

The following examples are provided for the purpose of illustrating the present invention but without limiting the scope thereof.

### REFERENCE EXAMPLE 1 - POLYURETHANE FOAM K-FACTORS

This example further demonstrates the unexpected performance of blowing agents in accordance with the present invention as used in the production of polyurethane foams. Three appliance polyurethane foams are made, each one being formed using substantially the same materials, procedures and equipment, with the exception that different blowing agents are used. The polyol system is a commercially available, appliance-type formulation adapted for use with a liquid blowing agent. A foam machine is used to form the foam. The blowing agents are used in essentially equal molar concentrations. After formation, each foam is cut into samples suitable for measuring k-factors, which are found to be as indicated in the following Table 1B below. The blowing agent composition in weight percent on the basis of total blowing agent is disclosed in Table 1A below:

**TABLE 1A**

| Blowing Agent | A | B | C |
|---|---|---|---|
| | | | |
| HFO-1234ze* | 85 | 0 | 60 |
| HFC-245fa | 15 | 100 | 11 |
| Cyclopentane | 0 | 0 | 29 |
| *100% cis | | | |

**TABLE 1B**

| Mean Temperature °C (°F) | k-factor in mW.m⁻¹K⁻¹ (BTU in / hr ft²°F) | | |
|---|---|---|---|
| | A | B | C |
| 4 (40) | 16.7 (0.116) | 17.2 (0.119) | 16.7 (0.116) |
| 24 (75) | 18.9 (0.131) | 19.3 (0.134) | 19.0 (0.132) |
| 43 (110) | 21.1 (0.146) | 21.5 (0.149) | 21.3 (0.148) |

### EXAMPLE 2 - POLYURETHANE FOAM K-FACTORS

A further experiment was performed using the same polyol formulation and isocyanate as in Example 1. The foam is prepared by hand mix, The blowing agents consist of a compound in accordance with Formula II, namely, HFCO-1233zd (CF3CH=CHCl)* in about the same mole percentage of the foamable composition as the blowing agent in Example 1. K-factors are found to be as indicated in Table 2 below.

**TABLE 2**

| Mean Temperature °C(°F) | k-factor mW.m⁻¹K⁻¹ (BTU in / hr ft²°F) |
|---|---|
| 4 (40) | 18.3 (0.127) |
| 24 (75) | 20.6 (0.143) |
| 43 (110) | 22.9 (0.159) |

## Claims

1. A thermoset foam comprising a plurality of polymeric cells and a blowing agent composition contained in at least one of said cells, said composition comprising HFCO-1233zd (1-chloro-3,3,3-trifluoropropene),
wherein the foam has a K factor (mW.m⁻¹K⁻¹((BTU in/hr ft² °F))) at 4°C (40 °F) of not greater than 20.2 (0.14)
wherein the HFCO-1233zd is present in the composition in an amount of at least 1% by weight of the composition and
wherein the composition has a Global Warming Potential (GWP) of not greater than 500.

2. The foam of claim 1, wherein the foam is a polyurethane foam, a polyisocyanurate foam or a phenolic foam.

3. The foam of claim 1 or 2, which has a K-factor (mW.m⁻¹K⁻¹((BTU/in hr ft² °F))) at 24°C (75°F) of not greater than 23.1 (0.16).

4. The foam of any of claims 1 to 3 wherein the foam is a refrigerator foam, a freezer foam, a refrigerator/freezer foam or a panel foam.

5. The foam of any one of claims 1 to 4 wherein said HFCO-1233zd comprises cis HFCO-1233zd and/or trans HFCO-1233zd.

6. The foam of any one of claims 1 to 5 wherein said HFCO-1233zd comprises trans-1233zd.

7. The foam of any one of claims 1 to 6 wherein the blowing agent further comprises at least one co-blowing agent selected from one or more of C1-C4 HFCs, C4-C6 hydrocarbons, water, CO₂, CFCs, HCCs, HCFCs, C1-C5 alcohols, C1-C4 aldehydes, C3-C4 ketones, C2-C4 ethers, methyl formate and combinations of two or more of these.

8. The foam of claim 7 wherein said one or more HFCs are selected from the group consisting of difluoromethane (HFC-32), fluoroethane (HFC-161) difluoroethane (HFC-152), trifluoroethane (HFC-143), tetrafluoroethane (HFC-134), pentafluoroethane (HFC-125), pentafluoropropane (HFC-245), hexafluoropropane (HFC-236), heptafluoropropane (HFC-227ea), pentafluorobutane (HFC-365), hexafluorobutane (HFC-356), all isomers of all of these, and combinations of two or more of these.

9. The foam of claim 1 wherein the composition further comprises methyl formate.

10. The foam of claim 7, wherein the co-blowing agent is selected from the group consisting of iso, normal and cyclopentane.

11. The foam of any preceding claim wherein the HFCO-1233zd is present in the composition in an amount of at least 5% by weight of the composition.

12. The foam of any preceding claim wherein the HFCO-1233zd is present in the composition in an amount of at least 15% by weight of the composition.

13. The foam of any preceding claim wherein HFCO-1233zd is present in the composition in an amount of at least 50% by weight of the composition.

14. The foam of any preceding claim wherein the blowing agent composition has a Global Warming Potential (GWP) of not greater than 150.

15. The foam of any preceding claim wherein the blowing agent composition has a Global Warming Potential (GWP) of not greater than 75.

16. The foam of any preceding claim wherein the blowing agent composition has a Ozone Depleting Potential (ODP) of not greater than 0.05.

17. The foam of any preceding claim wherein the blowing agent composition has a Ozone Depleting Potential (ODP) of not greater than 0.02

18. The foam of any preceding claim wherein the blowing agent composition has a Ozone Depleting Potential (ODP) of not greater than zero.

19. Use of a foam as defined in any preceding claim as an appliance foam selected from refrigerator foams, freezer foams, refrigerator/freezer foams, and panel foams.

20. A method of providing a thermoset foam according to any of claims 1 to 18, said method comprises (a) providing an A side component comprising an isocyanate and a B side component comprising a polyol or polyol mixture, a surfactant, a catalyst, a flame retardant and a composition comprising at least 1% by weight of HFCO-1233zd (1-chloro-3,3,3-trifluoropropene) and (b) mixing the A and B side components by hand mixing or machine mixing to form a foam.

21. A product selected from blocks, slabs, laminates, pour-in-place panels, spray-applied foam and froths, wherein said product comprises a foam obtainable from the method of claim 20.

## Patentansprüche

1. Wärmehärtender Schaumstoff, der eine Vielzahl von Polymerzellen und eine Blähmittelzusammensetzung in wenigstens einer der Zellen enthalten umfasst, wobei die Zusammensetzung HFCO-1233zd (1-Chlor-3,3,3-trifluorpropen) umfasst,
wobei der Schaumstoff einen K-Faktor (mW.m⁻¹K⁻¹ ((BTU in/h ft² °F))) bei 4 °C (40 °F) von nicht größer als 20,2 (0,14) aufweist,
wobei das HFCO-1233zd in der Zusammensetzung in einer Menge von wenigstens 1 Gew.-% der Zusammensetzung vorhanden ist und
wobei die Zusammensetzung ein Erderwärmungspotenzial (GWP-Wert) von nicht größer als 500 aufweist.

2. Schaumstoff gemäß Anspruch 1, wobei der Schaumstoff ein Polyurethanschaumstoff, ein Polyisocyanuratschaumstoff oder ein Phenolschaumstoff ist.

3. Schaumstoff gemäß Anspruch 1 oder 2, der einen K-Faktor (mW.m⁻¹K⁻¹ ((BTU in/h ft² °F))) bei 24 °C (75 °F) von nicht größer als 23,1 (0,16) aufweist.

4. Schaumstoff gemäß einem der Ansprüche 1 bis 3, wobei der Schamstoff ein Kühlschrank-Schaumstoff, ein Gefrierschrank-Schaumstoff, ein Kühl/Gefrierschrank-Schaumstoff oder ein Plattenschaumstoff ist.

5. Schaumstoff gemäß einem der Ansprüche 1 bis 4, wobei das HFCO-1233zd cis-HFCO-1233zd und/oder trans-HFCO-1233zd umfasst.

6. Schaumstoff gemäß einem der Ansprüche 1 bis 5, wobei das HFCO-1233zd trans-1233zd umfasst.

7. Schaumstoff gemäß einem der Ansprüche 1 bis 6, wobei das Blähmittel ferner wenigstens ein Co-Blähmittel ausgewählt aus einem oder mehreren von C1-C4-HFCs, C4-C6-Kohlenwasserstoffen, Wasser, CO₂, CFCs, HCCs, HCFCs, C1-C5-Alkoholen, C1-C4-Aldehyden, C3-C4-Ketonen, C2-C4-Ethern, Methylformiat und Kombinationen von zwei oder mehreren davon umfasst.

8. Schaumstoff gemäß Anspruch 7, wobei der eine oder die mehreren HFCs ausgewählt sind aus der Gruppe bestehend aus Difluormethan (HFC-32), Fluorethan (HFC-161), Difluorethan (HFC-152), Trifluorethan (HFC-143), Tetrafluorethan (HFC-134), Pentafluorethan (HFC-125), Pentafluorpropan (HFC-245), Hexafluorpropan (HFC-236), Heptafluorpropan (HFC-227ea), Pentafluorbutan (HFC-365), Hexafluorbutan (HFC-356), allen Isomeren von allen davon und Kombinationen von zwei oder mehreren davon.

9. Schaumstoff gemäß Anspruch 1, wobei die Zusammensetzung ferner Methylformiat umfasst.

10. Schaumstoff gemäß Anspruch 7, wobei das Co-Blähmittel ausgewählt ist aus der Gruppe bestehend aus iso-, normal- und Cyclopentan.

11. Schaumstoff gemäß einem der vorstehenden Ansprüche,
wobei das HFCO-1233zd in der Zusammensetzung in einer Menge von wenigstens 5 Gew.-% der Zusammensetzung vorhanden ist.

12. Schaumstoff gemäß einem der vorstehenden Ansprüche,
wobei das HFCO-1233zd in der Zusammensetzung in einer Menge von wenigstens 15 Gew.-% der Zusammensetzung vorhanden ist.

13. Schaumstoff gemäß einem der vorstehenden Ansprüche,
wobei das HFCO-1233zd in der Zusammensetzung in einer Menge von wenigstens 50 Gew.-% der Zusammensetzung vorhanden ist.

14. Schaumstoff gemäß einem der vorstehenden Ansprüche,
wobei die Blähmittelzusammensetzung ein Erderwärmungspotenzial (GWP-Wert) von nicht größer als 150 aufweist.

15. Schaumstoff gemäß einem der vorstehenden Ansprüche,
wobei die Blähmittelzusammensetzung ein Erderwärmungspotenzial (GWP-Wert) von nicht größer als 75 aufweist.

16. Schaumstoff gemäß einem der vorstehenden Ansprüche, wobei die Blähmittelzusammensetzung ein Ozonabbaupotenzial (ODP-Wert) von nicht größer als 0,05 aufweist.

17. Schaumstoff gemäß einem der vorstehenden Ansprüche,
wobei die Blähmittelzusammensetzung ein Ozonabbaupotenzial (ODP-Wert) von nicht größer als 0,02 aufweist.

18. Schaumstoff gemäß einem der vorstehenden Ansprüche,
wobei die Blähmittelzusammensetzung ein Ozonabbaupotenzial (ODP-Wert) von nicht größer als null aufweist.

19. Verwendung eines Schaumstoffs gemäß einem der vorstehenden Ansprüche als Geräteschaumstoff ausgewählt aus Kühlschrank-Schaumstoffen, Gefrierschrank-Schaumstoffen, Kühl/Gefrierschrank-Schaumstoffen und Plattenschaumstoffen.

20. Verfahren zum Bereitstellen eines wärmehärtenden Schaumstoffs gemäß einem der Ansprüche 1 bis 18,
wobei das Verfahren umfasst: (a) Bereitstellen einer Seitenkomponente A, die ein Isocyanat umfasst, und einer Seitenkomponente B, die ein Polyol oder Polyolgemisch, ein grenzflächenaktives Mittel, einen Katalysator, einen Flammhemmer und eine Zusammensetzung umfassend wenigstens 1 Gew.-% HFCO-1233zd (1-Chlor-3,3,3-trifluorpropen) umfasst, und (b) Mischen der Seitenkomponenten A und B durch Handmischen oder Maschinenmischen, um einen Schaumstoff zu bilden.

21. Produkt ausgewählt aus Blöcken, Platten, Laminaten, Pour-in-Place-Platten, aufgesprühtem Schaumstoff und Schäumen, wobei das Produkt einen durch das Verfahren gemäß Anspruch 20 erhältlichen Schaumstoff umfasst.

## Revendications

1. Mousse thermodurcie comprenant une pluralité de cellules polymères et une composition d'agent d'expansion contenue dans au moins une desdites cellules, ladite composition comprenant du HFCO-1233zd (1-chloro-3,3,3-trifluoropropène),
la mousse ayant un facteur K (mW.m⁻¹K⁻¹ ((BTU in/h ft²°F))) à 4 °C (40 °F) ne dépassant pas 20,2 (0,14),
le HFCO-1233zd étant présent dans la composition dans une quantité d'au moins 1 % en poids de la composition et
la composition ayant un potentiel de réchauffement global (PRG) ne dépassant pas 500.

2. Mousse de la revendication 1, la mousse étant une mousse de polyuréthane, une mousse de polyisocyanurate ou une mousse phénolique.

3. Mousse de la revendication 1 ou 2, qui a un facteur K (mW.m⁻¹K⁻¹ ((BTU/in h ft² °F))) à 24 °C (75 °F) ne dépassant pas 23,1 (0,16).

4. Mousse de l'une quelconque des revendications 1 à 3, la mousse étant une mousse pour réfrigérateurs, une mousse pour congélateurs, une mousse pour réfrigérateurs/congélateurs ou une mousse pour panneaux.

5. Mousse de l'une quelconque des revendications 1 à 4 dans laquelle ledit HFCO-1233zd comprend du cis-HFCO-1233zd et/ou du trans-HFCO-1233zd.

6. Mousse de l'une quelconque des revendications 1 à 5 dans laquelle ledit HFCO-1233zd comprend du trans-1233zd.

7. Mousse de l'une quelconque des revendications 1 à 6 dans laquelle l'agent d'expansion comprend en outre au moins un co-agent d'expansion choisi à partir d'un ou plusieurs composés parmi les HFC en C1-C4, les hydrocarbures en C4-C6, l'eau, le CO₂, les CFC, les HCC, les HCFC, les alcools en C1-C5, les aldéhydes en C1-C4, les cétones en C3-C4, les éthers en C2-C4, le formate de méthyle et les combinaisons d'au moins deux d'entre eux.

8. Mousse de la revendication 7 dans laquelle ledit ou lesdits HFC sont choisis dans le groupe constitué par le difluorométhane (HFC-32), le fluoroéthane (HFC-161), le difluoroéthane (HFC-152), le trifluoroéthane (HFC-143), le tétrafluoroéthane (HFC-134), le pentafluoroéthane (HFC-125), le pentafluoropropane (HFC-245), l'hexafluoropropane (HFC-236), l'heptafluoropropane (HFC-227ea), le pentafluorobutane (HFC-365), l'hexafluorobutane (HFC-356), tous les isomères de tous ceux-ci, et les combinaisons d'au moins deux d'entre eux.

9. Mousse de la revendication 1 dans laquelle la composition comprend en outre du formate de méthyle.

10. Mousse de la revendication 7, dans laquelle le co-agent d'expansion est choisi dans le groupe constitué par l'isopentane, le pentane normal et le cyclopentane.

11. Mousse d'une quelconque revendication précédente dans laquelle le HFCO-1233zd est présent dans la composition dans une quantité d'au moins 5 % en poids de la composition.

12. Mousse d'une quelconque revendication précédente dans laquelle le HFCO-1233zd est présent dans la composition dans une quantité d'au moins 15 % en poids de la composition.

13. Mousse d'une quelconque revendication précédente dans laquelle le HFCO-1233zd est présent dans la composition dans une quantité d'au moins 50 % en poids de la composition.

14. Mousse d'une quelconque revendication précédente dans laquelle la composition d'agent d'expansion a un potentiel de réchauffement global (PRG) ne dépassant pas 150.

15. Mousse d'une quelconque revendication précédente dans laquelle la composition d'agent d'expansion a un potentiel de réchauffement global (PRG) ne dépassant pas 75.

16. Mousse d'une quelconque revendication précédente dans laquelle la composition d'agent d'expansion a un potentiel d'appauvrissement de la couche d'ozone (PACO) ne dépassant pas 0,05.

17. Mousse d'une quelconque revendication précédente dans laquelle la composition d'agent d'expansion a un potentiel d'appauvrissement de la couche d'ozone (PACO) ne dépassant pas 0,02.

18. Mousse d'une quelconque revendication précédente dans laquelle la composition d'agent d'expansion a un potentiel d'appauvrissement de la couche d'ozone (PACO) ne dépassant pas zéro.

19. Utilisation d'une mousse telle que définie dans une quelconque revendication précédente comme une mousse pour appareils ménagers choisie parmi les mousses pour réfrigérateurs, les mousses pour congélateurs, les mousses pour réfrigérateurs/congélateurs, et les mousses pour panneaux.

20. Procédé d'obtention d'une mousse thermodurcie selon l'une quelconque des revendications 1 à 18, ledit procédé comprenant (a) l'obtention d'un composant côté A comprenant un isocyanate et d'un composant côté B comprenant un polyol ou un mélange de polyols, un tensioactif, un catalyseur, un ignifugeant et une composition comprenant au moins 1 % en poids de HFCO-1233zd (1-chloro-3,3,3-trifluoropropène) et (b) le mélange des composants des côtés A et B par mélange à la main ou mélange en machine pour former une mousse.

21. Produit choisi parmi des blocs, des dalles, des stratifiés, des panneaux coulés sur place, de la mousse ou des mousses appliquées par projection, ledit produit comprenant une mousse pouvant être obtenue par le procédé de la revendication 20.
